# FASCICULE DE BREVET EUROPEEN

(11) **EP 2 498 901 B1**
(45) Date de publication et mention de la délivrance du brevet: **26.03.2014**
(21) Numéro de dépôt: 10795427.3
(22) Date de dépôt: 10.11.2010
(51) Int. Cl.: B01F 11/00, C12M 3/06, B01L 9/06, B01F 13/10, B01F 15/00

(54) **APPAREIL POUR LA VIBRATION RAPIDE DE TUBES CONTENANT EN PARTICULIER DES ECHANTILLONS BIOLOGIQUES**
VORRICHTUNG ZUM SCHNELLEN VIBRIEREN VON PROBERÖHRCHEN ENTHALTEND INSBESONDERE BIOLOGISCHE PROBEN
DEVICE FOR RAPID VIBRATING OF TUBES CONTAINING PARTICULARLY BIOLOGICAL SAMPLES

(30) Priorité: 10.11.2009 FR 0905389
(43) Date de publication de la demande: 19.09.2012
(73) Titulaire: Bertin Technologies, 78180 Montigny-le-Bretonneux (FR)
(72) Inventeur: BOQUET, Jean, F-78610 Le Perray en Yvelines (FR)
(74) Mandataire: Ramey, Daniel
(86) Numéro de dépôt international: PCT/FR2010/052414
(87) Numéro de publication internationale: WO 2011/058278

(56) Documents cités:
- WO-A1-2004/105925
- WO-A2-2008/000962
- FR-A1- 2 839 267
- GB-A- 684 615
- US-A- 5 567 050
- US-A- 5 707 861
- US-B1- 6 235 501

## Description

L'invention concerne un appareil pour la vibration rapide de tubes contenant des échantillons, en particulier des échantillons biologiques, la vibration rapide des tubes réalisant un broyage, une agitation et/ou une homogénéisation des échantillons.

Il est déjà connu de broyer des échantillons biologiques (tissus, céréales, cheveux, etc.) en enfermant les échantillons dans des tubes qui contiennent également des microbilles de verre, de céramique, d'acier ou autre et en soumettant les tubes fermés de façon étanche à une vibration essentiellement axiale à fréquence élevée, par exemple d'environ 100 Hz, pendant une durée relativement courte de l'ordre de 30 à 60 secondes par exemple.

Différents documents tels que US-A-5 567 050 et W02004012851 décrivent des appareils pour l'exécution d'un tel procédé. Ils comprennent un plateau circulaire de support des tubes et des moyens d'entraînement du plateau en mouvement oscillatoire autour d'un centre de rotation.

Les tubes fixés à la périphérie du plateau à égale distance du centre de rotation sont ainsi déplacés en mouvement alternatif sensiblement curviligne.

Théoriquement, la vitesse de rotation de l'arbre de sortie du moteur est comprise entre 3000 et 8000 tours par minute et les échantillons sont soumis à des accélérations linéaires de 150 à 400g, pour leur broyage.

Mais ces appareils travaillent sur un grand nombre d'échantillons simultanément (en général 12, 16, 24 ou 48 échantillons).

Ces appareils sont destinés à de gros laboratoires qui doivent analyser de façon continue une grande quantité d'échantillons et en général selon un même protocole. Un exemple typique est donné par les laboratoires intégrés aux abattoirs de bovins pour la détection de l'ESB. Ces laboratoires doivent analyser un échantillon de cerveau de chaque animal et donner le résultat de l'analyse avant la sortie de la carcasse de l'abattoir. Cela représente plus de mille analyses par jour.

Beaucoup de laboratoires n'ont pas une telle spécificité. Ils peuvent n'avoir besoin d'un broyage ou d'une agitation d'échantillon qu'une ou deux fois par jour et même moins, ou doivent travailler successivement sur des échantillons différents nécessitant des protocoles différents.

Ces laboratoires peuvent également devoir travailler sur des échantillons de volumes différents, par conséquent contenus dans des tubes de tailles différentes, par exemple de 2 ml et 7 ml.

Pour ces très nombreux laboratoires, l'utilisation d'une machine de 16 ou 24 tubes est possible mais non rentable.

La présente invention a notamment pour but d'apporter une solution simple, efficace et économique à ce problème.

Elle a pour objet un appareil du type précité, qui permet la vibration rapide de quelques tubes contenant des échantillons à analyser avec une qualité et une efficacité au moins équivalentes à celles des appareils connus.

Elle a également pour objet un appareil qui peut fonctionner sans dommage à des fréquences élevées.

Elle a encore pour objet un appareil de petite dimension, simple d'usage et d'entretien, qui peut facilement trouver sa place sur une paillasse de laboratoire.

Elle propose à cet effet un appareil pour la vibration rapide de tubes contenant des échantillons, en particulier biologiques, comprenant un moteur électrique d'entrainement en rotation d'un disque muni d'un axe excentré, caractérisé en ce que les tubes sont portés par un support sensiblement parallèle à l'axe excentré et sont orientés sensiblement perpendiculairement à l'axe excentré, qui est relié à un châssis fixe par un centre d'articulation à deux axes de rotation perpendiculaires entre eux et dont l'un est sensiblement parallèle à l'axe excentré et relie le support à l'autre axe du centre d'articulation qui bloque la rotation du support autour d'un axe perpendiculaire aux deux axes précités.

L'appareil selon l'invention comprend donc un support des tubes et des moyens d'entrainement du support pour réaliser un mouvement oscillatoire autour d'un centre d'articulation, le support des tubes étant lié à une partie fixe de l'appareil par une liaison à deux degrés orthogonaux de liberté en rotation, autour d'axes X et Y d'un repère orthonormé, permettant le mouvement oscillatoire dudit support autour du centre d'articulation tout en restant à une distance constante de ce centre d'articulation.

Le mouvement de vibration du support des tubes, généré par un disque tournant comportant un axe excentré, est transmis audit support par une biellette fixée sur celui-ci par une chape dont l'axe d'articulation est transversal ou orthogonal à la longueur du support. Le mouvement transmis aux tubes comportant les échantillons est alors une trajectoire en forme de 8 sur une surface sensiblement sphérique. Cette trajectoire a la particularité de produire un balayage complet de l'intérieur des tubes par les billes et réaliser un broyage efficace des échantillons.

De préférence, le centre d'articulation du support des tubes est matérialisé par une articulation de type cardan, une chape, une bande d'élastomère ou de polymère ou une rangée de plots en élastomère.

La biellette est reliée à l'axe du disque tournant par un roulement à billes rotulant. Sa chape de fixation sur le support lui permet de s'adapter aux déformations dues aux mouvements relatifs entre l'axe du disque d'entrainement et le support. Sa longueur efficace est sensiblement identique à la distance entre son point de fixation sur le support et le centre d'articulation du support sur l'appareil.

Dans un mode de réalisation, l'ensemble centre d'articulation, support et biellette est réalisé en une seule pièce de métal, d'élastomère ou de polymère. Des inserts, introduits au moulage ou coulage de la pièce, permettent d'assurer une meilleure rigidité en rotation autour d'un axe Z tout en permettant les mouvements autour des axes X et Y et en assurant également la légère capacité d'adaptation de la biellette à la trajectoire de l'axe du disque tournant.

Selon d'autres caractéristiques de l'invention, les tubes sont maintenus sur le support à l'aide de pinces. Avantageusement, ils pourront être légèrement inclinés pour favoriser un balayage total, par les billes, du volume interne des tubes.

Dans une définition de base, le support est équipé d'un ensemble de pinces capable de recevoir un, deux ou trois tubes de petit volume, par exemple de 2ml et/ou un tube de grand volume par exemple 7ml.

Dans un mode de réalisation préféré, le disque tournant est entrainé en rotation par un moteur électrique dont l'utilisateur peut régler la vitesse de rotation, par exemple de 3000 à 7000 tours/minute et la durée de fonctionnement, par exemple de 30 à 180 secondes.

Dans deux variantes offrant le même fonctionnement, la biellette, le disque tournant et le moteur sont placés au dessous ou au dessus du support des tubes.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :
- la figure 1 est une vue schématique en perspective d'un mode de réalisation d'un appareil selon l'invention, sans habillage;
- la figure 2 est une vue schématique en perspective de l'ensemble centre d'articulation, support, biellette, pinces à tubes;
- les figures 3a et 3b sont des vues de face et de côté d'un exemple d'aménagement des composants et de réalisation de l'appareil ;
- les figures 4 et 5 sont des vues schématiques en perspective de variantes de réalisation ;
- les figures 6a et 6b sont des vues schématiques en perspective de variantes de réalisation de cales de support et de positionnement des tubes.

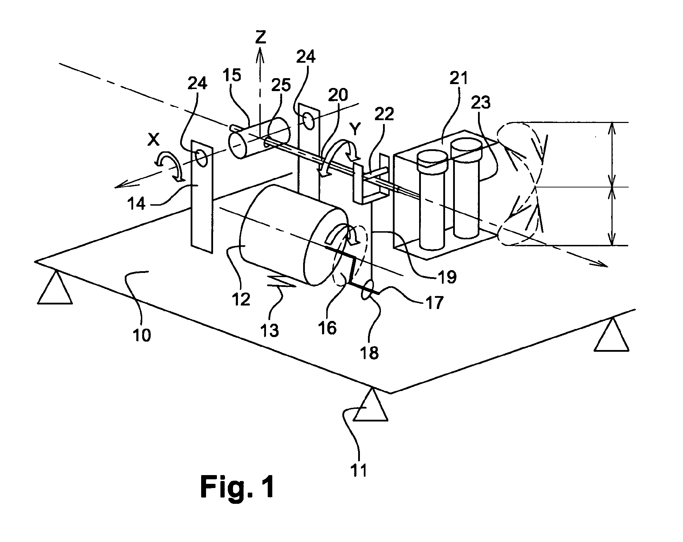

Dans le mode de réalisation de la figure 1, l'appareil selon l'invention comprend un châssis 10 dont la partie inférieure est munie de pieds isolants 11 pour reposer sur une paillasse de laboratoire ou un autre support fixe plan et dont la partie supérieure reçoit un moteur électrique 12 par l'intermédiaire d'une liaison 13 filtrant les vibrations et un support 14 d'un centre d'articulation 15.

Un disque 16, fixé sur l'arbre du moteur 12, est muni d'un axe excentré 17 sur lequel vient se fixer une biellette 19 par l'intermédiaire d'un roulement rotulant 18.

La biellette 19 est fixée à un support 20 par l'intermédiaire d'une chape 22 capable d'imposer au support 20 des mouvements de rotation autour de deux axes X et Y perpendiculaires dont l'intersection définit le centre d'articulation 15.

L'articulation 15 est fixée au support 14 à l'aide de paliers 24 de préférence à billes assurant un blocage axial et une seule liberté en rotation autour de l'axe X.

Le support 20 est fixé à l'articulation 15 par un palier 25 de préférence à billes ou à rouleaux assurant un blocage axial et une seule liberté en rotation autour de l'axe Y.

Une pince 21 de réception et de maintien des tubes 23 est fixée rigidement au support 20. Elle peut recevoir un petit nombre de tubes, compris entre 1 et 5 par exemple.

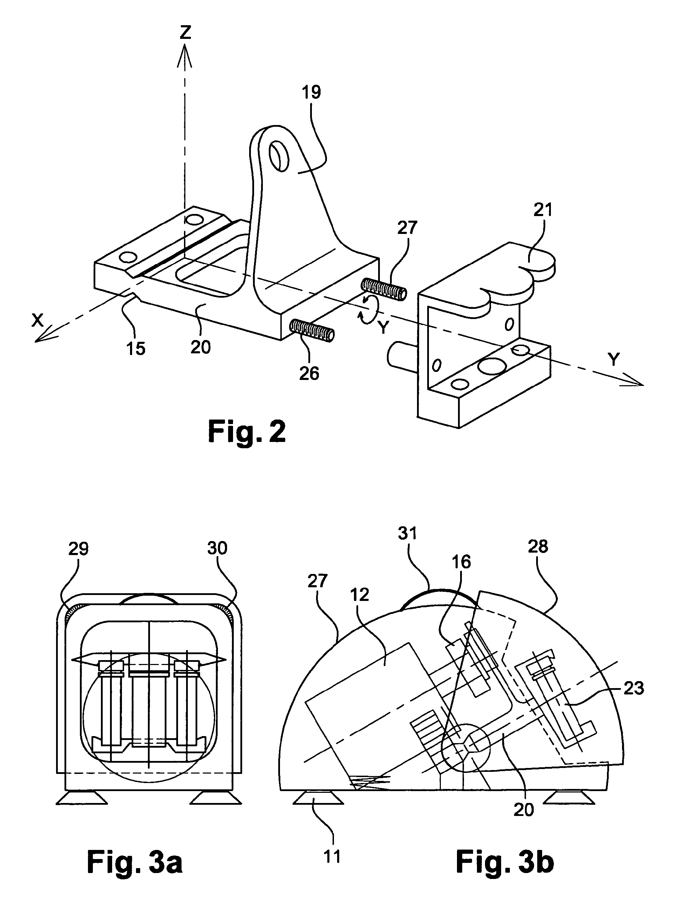

Dans le mode de réalisation représenté en figure 2, le support 20, le centre d'articulation 15, la biellette 19 sont réalisés en une seule pièce en polymère ou élastomère. Des raidisseurs 26 sont insérés dans le support pour favoriser les articulations autour des axes X et Y du centre d'articulation 15 et bloquer les rotations autour de l'axe Z perpendiculaire aux axes X et Y. Le matériau constituant le support est choisi pour offrir une grande résistance aux flexions successives de l'articulation 15.

Les grands avantages de ce mode de réalisation sont le faible coût de réalisation et d'entretien et l'importante réduction du niveau de bruit du à l'absence de pièces mécaniques.

Le mode de réalisation représenté aux figures 3a et 3b utilise le support 20 de la figure 2. Le moteur 12 est placé en oblique au dessus du support 20 et des tubes 23 qui sont inclinés par rapport à la verticale. Cet agencement permet de réaliser un appareil compact et ergonomique. L'ensemble est placé dans un capotage 27 comportant une visière 28. Le basculement de la visière 28 vers l'arrière donne l'accès aux tubes 23 et aux molettes 29, 30 de réglage de la vitesse de rotation du moteur et de la durée de fonctionnement. En outre il interdit l'accès au bouton 31 de mise en marche/arrêt du moteur.

Le basculement de la visière vers l'avant, protège l'opérateur et l'environnement, empêche l'accès aux tubes 23, aux molettes 29 et 30 et donc empêche la modification du réglage des paramètres de fonctionnement et autorise l'accès au bouton 31 pour la mise en route/arrêt prématuré de l'appareil.

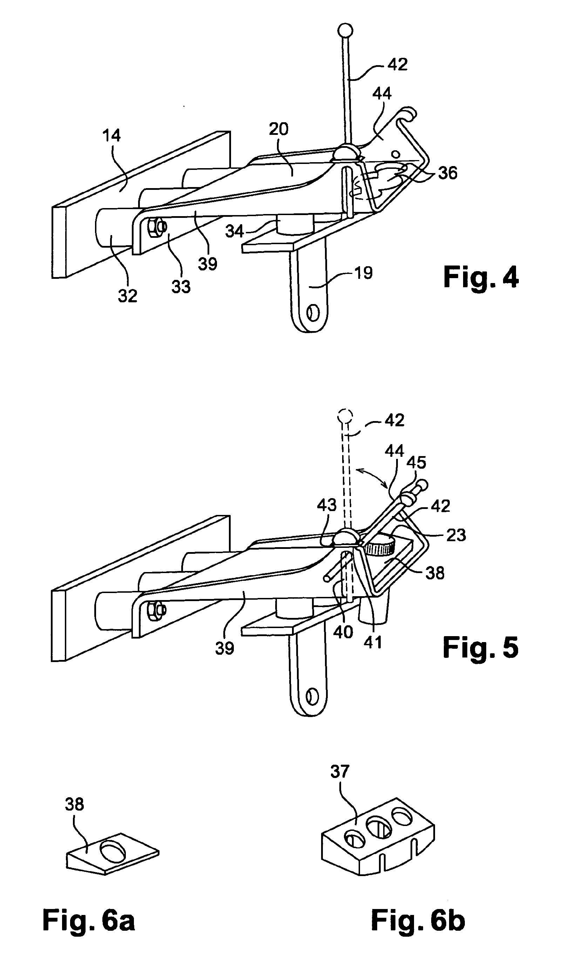

Dans le mode de réalisation représenté en figure 4, le support 20 et la pince 21 de réception des tubes 23 sont réalisés en une seule plaque de métal léger ou de matériau plastique rigide constitué d'un plat, d'un bord en équerre 33 et de deux flancs raidisseurs 39 et 44. Le centre d'articulation 15 est constitué d'une rangée de trois plots en élastomère 32 fixés par vissage ou vulcanisation d'un coté sur un support fixe 14 et de l'autre coté sur le bord en équerre 33 du support 20. Ces plots sont parallèles entre eux et à l'axe Y. en variante, les deux plots extérieurs peuvent parallèles à l'axe Y et le plot central parallèle à l'axe Z, leurs centres de rotation étant alignés sur l'axe X afin de favoriser les rotations du support 20 autour des axes X et Y et limiter les déplacements par rapport à l'axe Z.

La biellette 19 est fixée au support 20 par l'intermédiaire d'une rangée de trois plots en élastomère 34 fixés par vissage ou vulcanisation d'un côté sur le support 20 et de l'autre côté sur une partie en équerre de la biellette 19.

Les tubes 23 de 2 ml ou 7 ml sont engagés et maintenus dans des trous 36 formés à l'avant du support 20.

Dans le mode de réalisation représenté aux figures 5, 6a et 6b, les tubes 23 de 2 ml ou 7 ml sont positionnés et maintenus dans les trous 36 par l'intermédiaire de cales 37 ou 38 qui sont avantageusement biaises pour incliner légèrement les tubes par rapport au plan du support 20. La cale 37 est adaptée à recevoir indifféremment un, deux ou trois tubes de 2 ml. La cale 38 est adaptée à recevoir un tube de 7 ml.

Un des flancs 39 du support 20 comporte une fente 40 et des encoches 41. Une tige 42 en fibre de verre ou de carbone par exemple est munie d'un anneau en élastomère 43. L'anneau 43 est positionné dans les encoches 41 et la tige 42 dans la fente 40 de telle sorte que la tige 42 est maintenue en position verticale grâce à l'anneau 43.

L'autre flanc 44 comporte un crochet 45 pour recevoir l'autre extrémité de la tige 42.

Le ou les tubes peuvent être bloqués sur le support 20 par l'intermédiaire des cales 37 ou 38 et de la tige 42 qui s'incurve entre la fente 40 et le crochet 45.

Dans le mode de réalisation de la figure 6b, pour garantir un serrage uniforme dans le cas de l'utilisation de deux ou trois tubes de 2 ml, la cale 37 est fendue entre les orifices de réception des tubes pour permettre sa déformation et son auto adaptation aux éventuelles variations de hauteur des tubes. Avantageusement la déformation des extrémités de la cale provoque une mise en convergence des tubes d'extrémité favorable à l'homogénéité de la qualité des broyages dans les différents tubes. Une cale 37 pourrait également être conçue pour recevoir deux tubes de 2 ml sur les extrémités et un tube de 7 ml au centre.

## Revendications

1. Appareil pour la vibration rapide de tubes contenant des échantillons à broyer, comprenant un moteur électrique (12) d'entrainement en rotation d'un disque (16) muni d'un axe excentré (17), **caractérisé en ce que** les tubes (23) sont portés par un support (20) sensiblement parallèle à l'axe excentré et sont orientés sensiblement perpendiculairement à l'axe excentré (17), qui est relié à un châssis fixe par un centre d'articulation (15) à deux axes de rotation (X, Y), perpendiculaires entre eux et dont l'un (Y) est sensiblement parallèle à l'axe excentré (17) et relie le support (20) à l'autre axe (X) du centre d'articulation (15) qui bloque la rotation du support (20) autour d'un axe (Z) perpendiculaire aux deux axes précités (X, Y).

2. Appareil selon la revendication 1, **caractérisé en ce que** le support (20) est relié à l'axe excentré (17) par une biellette perpendiculaire (19) dont une extrémité est fixée sur le support (20) par une liaison (22) de type chape et dont l'autre extrémité comprend une liaison (18) articulée sur l'axe excentré (17).

3. Appareil selon la revendication 2, **caractérisé en ce que** la liaison (18) est un roulement à billes rotulant.

4. Appareil selon l'une des revendications 1 à 3, **caractérisé en ce que** la rotation du disque (16) entraine un mouvement de vibration des tubes (23) autour de l'axe (X) associé à un mouvement de rotation autour de l'axe (Y).

5. Appareil selon la revendication 2 ou 3, **caractérisé en ce que** la biellette (19) et l'articulation (22) du type chape sont réalisés d'une pièce en métal ou en un matériau élastiquement déformable tel qu'un polymère ou un élastomère.

6. Appareil selon l'une des revendications 2 à 5, **caractérisé en ce que** le support (20) est réalisé d'une seule pièce avec la biellette (19) et l'articulation (22) du type chape.

7. Appareil selon l'une des revendications 2 à 5, **caractérisé en ce que** le support (20) est réalisé d'une seule pièce avec la biellette (19), l'articulation (22) du type chape et le centre d'articulation (15).

8. Appareil selon l'une des revendications 1 à 7, **caractérisé en ce que** le centre d'articulation (15) est formé par une articulation de type cardan, par une chape, par une bande d'élastomère ou de polymère ou par une rangée de plots en élastomère (32).

9. Appareil selon l'une des revendications 1 à 8, **caractérisé en ce que** le moteur (12) et l'axe excentré (17) s'étendent en oblique au-dessus du support (20) et des tubes (23) qui sont inclinés par rapport à la verticale.

10. Appareil selon l'une des revendications 1 à 9, **caractérisé en ce que** le support (20) porte un petit nombre de tubes (23), compris entre 1 et 5 par exemple.

11. Appareil selon l'une des revendications 1 à 10, **caractérisé en ce que** la vitesse de rotation du disque (16) portant l'axe excentré (17) est comprise entre 3000 et 7000 tours / minute.

## Patentansprüche

1. Vorrichtung zum schnellen Vibrieren von Röhrchen mit zu zerstoßenden Proben, enthaltend einen Elektromotor (12) zum rotierenden Antreiben einer Scheibe (16), die mit einer exzentrischen Achse (17) versehen ist, **dadurch gekennzeichnet, dass** die Röhrchen (23) von einem im Wesentlichen parallel zur exzentrischen Achse verlaufenden Träger (20) getragen werden und im Wesentlichen senkrecht zur exzentrischen Achse (17) ausgerichtet sind, der mit einem festen Gestell über eine Anlenkstelle (15) mit zwei Drehachsen (X, Y) verbunden ist, die senkrecht zueinander verlaufen und von denen die eine (Y) im Wesentlichen parallel zur exzentrischen Achse (17) verläuft und den Träger (20) mit der anderen Achse (X) der Anlenkstelle (15) verbindet, welche die Drehung des Trägers (20) um eine senkrecht zu den beiden vorgenannten Achsen (X, Y) verlaufende Achse (Z) blockiert.

2. Vorrichtung nach Anspruch 1, **dadurch gekennzeichnet, dass** der Träger (20) mit der exzentrischen Achse (17) über einen senkrecht verlaufenden Schwenkarm (19) verbunden ist, von dem ein Ende über eine gabelartige Verbindung (22) an den Träger (20) befestigt ist und von dem das andere Ende eine an die exzentrische Achse (17) angelenkte Verbindung (18) aufweist.

3. Vorrichtung nach Anspruch 2, **dadurch gekennzeichnet, dass** die Verbindung (18) ein Pendelkugellager ist.

4. Vorrichtung nach einem der Ansprüche 1 bis 3, **dadurch gekennzeichnet, dass** die Drehung der Scheibe (16) eine Vibrationsbewegung der Röhrchen (23) um die Achse (X) herum bewirkt, mit der eine Drehbewegung um die Achse (Y) einhergeht.

5. Vorrichtung nach Anspruch 2 oder 3, **dadurch gekennzeichnet, dass** der Schwenkarm (19) und die gabelartige Anlenkung (22) aus einem Teil aus Metall oder einem elastisch verformbaren Material, wie etwa ein Polymer oder ein Elastomer, hergestellt sind.

6. Vorrichtung nach einem der Ansprüche 2 bis 5, **dadurch gekennzeichnet, dass** der Träger (20) aus einem Stück mit dem Schwenkarm (19) und der gabelartigen Anlenkung (22) hergestellt ist.

7. Vorrichtung nach einem der Ansprüche 2 bis 5, **dadurch gekennzeichnet, dass** der Träger (20) aus einem Stück mit dem Schwenkarm (19), der gabelartigen Anlenkung (22) und der Anlenkstelle (15) ausgebildet ist.

8. Vorrichtung nach einem der Ansprüche 1 bis 7, **dadurch gekennzeichnet, dass** die Anlenkstelle (15) aus einem Kardangelenk, einem Gabelgelenk, einem Band aus Elastomer oder Polymer oder aus einer Reihe von Zapfen aus Elastomer (32) ausgebildet ist.

9. Vorrichtung nach einem der Ansprüche 1 bis 8, **dadurch gekennzeichnet, dass** der Motor (12) und die exzentrische Achse (17) sich schräg über dem Träger (20) und den Röhrchen (23) erstrecken, die zur Senkrechten geneigt verlaufen.

10. Vorrichtung nach einem der Ansprüche 1 bis 9, **dadurch gekennzeichnet, dass** der Träger (20) eine kleinere Anzahl von Röhrchen (23) trägt, die beispielsweise zwischen 1 und 5 liegt.

11. Vorrichtung nach einem der Ansprüche 1 bis 10, **dadurch gekennzeichnet, dass** die Drehzahl der die exzentrische Achse (17) tragenden Scheibe (16) zwischen 3000 und 7000 Umdrehungen/Minute liegt.

## Claims

1. An appliance for rapidly vibrating test tubes containing samples to be ground up, the appliance comprising an electric motor (12) for driving rotation of a disk (16) that is provided with an eccentric pin (17), the appliance being **characterized in that** the test tubes (23) are carried by a support (20) substantially parallel to the eccentric pin and are oriented substantially perpendicularly to the eccentric pin (17), which pin is connected to a fixed baseplate via a hinge center (15) having two mutually perpendicular axes of rotation (X, Y), one of which axes (Y) is substantially parallel to the eccentric pin (17) and connects the support (20) to the other axis (X) of the hinge center (15) that prevents the support (20) from moving in rotation about an axis (Z) perpendicular to the two above-mentioned axes (X, Y).

2. An appliance according to claim 1, **characterized in that** the support (20) is connected to the eccentric pin (17) by a perpendicular link (19) having one end fastened to the support (20) by a fork-type connection (22) and having its other end including a hinged connection (18) to the eccentric pin (17).

3. An appliance according to claim 2, **characterized in that** the connection (18) is a rolling ball joint.

4. An appliance according to any one of claims 1 to 3, **characterized in that** rotation of the disk (16) drives vibratory movement of the tubes (23) about the axis (X) associated with rotary movement about the axis (Y).

5. An appliance according to claim 2 or claim 3, **characterized in that** the link (19) and the fork-type hinge (22) are in the form of a part made of metal or of an elastically-deformable material such as a polymer or an elastomer.

6. An appliance according to any one of claims 2 to 5, **characterized in that** the support (20) is made as a single part together with the link (19) and the fork-type hinge (22).

7. An appliance according to any one of claims 2 to 5, **characterized in that** the support (20) is made as a single part together with the link (19), the fork-type hinge (22), and the hinge center (15).

8. An appliance according to any one of claims 1 to 7, **characterized in that** the hinge center (15) is formed by a Cardan type joint, by a fork, by a strip of elastomer or of polymer, or by a row of elastomer studs (32).

9. An appliance according to any one of claims 1 to 8, **characterized in that** the motor (12) and the eccentric pin (17) extend obliquely above the support (20) and the test tubes (23) that are inclined relative to the vertical.

10. An appliance according to any one of claims 1 to 9, **characterized in that** the support (20) carries a small number of test tubes (23) directly, the number lying in the range 1 to 5, for example.

11. An appliance according to any one of claims 1 to 10, **characterized in that** the speed of rotation of the disk (16) carrying the eccentric pin (17) lies in the range 3000 rpm to 7000 rpm.
